Europäisches Patentamt

(19) **European Patent Office**

Office européen des brevets

(11) Publication number: **0 206 611**
**B1**

(12) **EUROPEAN PATENT SPECIFICATION**

(45) Date of publication of the patent specification:
01.03.89

(51) Int. Cl.⁴: **C 07 C 43/23, C 07 C 41/01, C 07 C 41/24**

(21) Application number: **86304380.8**

(22) Date of filing: **09.06.86**

(54) 3-(4-Fluorophenoxy)-1-Propanol.

(30) Priority: **14.06.85 PC /US85/011 33**

(43) Date of publication of application:
**30.12.86 Bulletin 86/52**

(45) Publication of the grant of the patent:
**01.03.89 Bulletin 89/9**

(84) Designated Contracting States:
**BE CH DE FR GB IT LI LU NL SE**

(73) Proprietor: **PFIZER INC., 235 East 42nd Street, New York, N.Y. 10017 (US)**

(72) Inventor: **Moore, Bernard Shields, 39, Savi Avenue, Waterford Connecticut (US)**
Inventor: **Urban, Frank John, 12, Twin Lakes Drive, Waterford Connecticut (US)**

(74) Representative: **Graham, Philip Colin Christison et al, Patents Department Pfizer Limited Ramsgate Road, Sandwich, Kent CT13 9NJ (GB)**

(56) References cited:
DE-A- 2 165 431
FR-A- 2 389 594
US-A- 3 188 338

CHEMICAL ABSTRACTS, vol. 89, no. 19, 6th November 1978, page 108, column 2, abstract no. 158025j, Columbus, Ohio, US; P. GILBERT et al.: "The lethal action of 2-phenoxyethanol and its analogs on Escherichia coli NCTC 5933", & MICROBIOS 1977, 19(76), 125-143
CHEMICAL ABSTRACTS, vol. 68, no. 8, 19th February 1968, page 3240, column 1, abstract no. 33182t, Columbus, Ohio, US; F.M. BERGER et al.: "Reduction of hypersensitivity to penicillins", & GB - A - 1 094 083 (CARTER-WALLACE) 06-12-1967
CHEMICAL ABSTRACTS, vol. 80, no. 25, 24th June 1974,

(56) References cited: (continuation)
page 401, column 2, abstract no. 145636y, Columbus, Ohio, US; S.T. AKHMEDOV et al.: "Synthesis and reaction of beta-(substituted-phenoxy) propyl alcohols with aromatic isocyanates", & ZH. ORG. KHIM. 1974, 10(3), 486-488

ACTORUM AG

## Description

This invention relates to a new and useful intermediate in the overall production of an asymmetric hydantoin compound. More particularly, it is concerned with a novel compound, 3-(4-fluorophenoxy)-1-propanol, which is of value as an intermediate in the synthesis of 6-fluoro-4-chromanone. The latter product is a key intermediate in the production of (4S)-6-fluoro-spiro-[chroman-4,4'-imidazolidine]-2',5'-dione (sorbinol), which is of especial importance in the field of medicinal chemistry in view of its ability to act as in aldose reductase inhibitor and thereby effectively control certain chronic diabetic complications, such as diabetic cataracts and neuropathy, etc.

According to the prior art, sorbinil was first reported as d-6-fluoro-spiro-[chroman-4,4'-imidazolidine]-2',5'-dione by R. Sarges in U.S. Patent No. 4 130 714. It was originally prepared by a multistep process which essentially involved condensing 6-fluoro-4-chromanone with potassium cyanide and ammonium carbonate to form the corresponding racemic precursor, followed by resolution of the latter dl-compound with 1-brucine. The 6-fluoro-4-chromanone used in the process was ultimately derived from 4-fluorophenol in a series of steps which first involved converting the latter compound to 3-(4-fluorophenoxy)-propionic acid according to the method described by G.C. Finger et al. in the Journal of the American Chemical Society, vol. 81, p. 94 (1959), followed by intramolecular condensation of the latter intermediate acid in the presence of polyphosphoric acid to effect ring-closure to the desired chromanone compound.

Later developments in the overall method of production led to a process for producing sorbinil which involved the following steps: (1) 4-fluorophenol was first converted to 3-(4-fluorophenoxy)-propionitrile by treatment with acrylonitrile in the presence of Triton B; (2) the nitrile intermediate was then converted to 3-(4-fluorophenoxy)propionic acyd by means of hydrochloric acid; (3) 3-(4-fluorophenoxy)propionic acid was then condensed in the presence of concentrated sulfuric acid at 50°C to afford 6-fluoro-4-chromanone; (4) the latter compound was thereafter condensed with potassium cyanide and ammonium carbonate in ethanol/water under standard Bucherer-Berg conditions to give the racemic precursor of sorbinil, which is called dl-6-fluoro-spiro-[chroman-4,4'-imidazolidine]-2',5'-dione; (5) the latter racemic spiro-hydantoin was then hydrolyzed with aqueous sodium hydroxide to the corresponding spiro-amino acid, which is called 4-amino-6-fluorochroman-4-carboxylic acid; (6) the latter acid, which can not be conveniently isolated in the process, was then treated in situ with sodium or potassium cyanate (after first adjusting the pH of the aqueous solution) in order to convert the amino acid to the corresponding hydantoic acid, which is called 6-fluoro-4-ureidochroman-4-carboxylic acid; (7) the latter hydantoic acid was then resolved according to the method described by B. W. Cue, Jr. et al. in the U.S. Patent No. 4 435 578 by treatment with 1-(−)-ephedrine in aqueous methanol to form the 1-(−)-

ephedrine salt of (4S)-6-fluoro-4-ureidochroman-4-carboxylic acid; and (8) the latter crystalline salt was thereafter converted to sorbinil by heating the diastereoisomer in glacial acetic acid to effect conversion to the desired (4S)-6-fluoro-spiro-[chroman-4,4'-imidazolidine]-2',5'-dione.

However, in the continuing effort to still further improve the overall production of (4S)-6-fluoro-spiro-[chroman-4,4'-imidazolidine]-2',5'-dione by employing the eight-step method of synthesis just described, there is a need to alter some of the steps leading to the production of 6-fluoro-4-chromanone, particularly in view of the fact that acrylonitrile is employed as the reagent in step (1). In the latter connection, it should be noted that the acrylonitrile route definitely depends upon a reversible reaction which does not fully utilize the 4-fluorophenol starting material. Furthermore, acrylonitrile is an extremely hazardous reagent and requires special handling conditions which, of course, presents still another drawback for large-scale commercial production.

In accordance with the present invention, there is now provided a novel compound, viz., 3-(4-fluorophenoxy)-1-propanol, which has been prepared by reacting 4-fluorophenol with 1-chloro-3-hydroxypropane or 1-bromo-3-hydroxypropane. This novel product is useful as an intermediate in the synthesis of 6-fluoro-4-chromanone, which is itself a key intermediate in the production of (4S)-6-fluoro-spiro-[chroman-4,4'-imidazolidine]-2',5'-dione as hereinbefore discussed. More particularly, the synthesis and use of this novel intermediate is accomplished in such a way that the aforementioned drawbacks of the prior art with respect to step (1) in the overall method of synthesis for sorbinil are overcome. For instance, the initial alkylation reaction of 4-fluorophenol with 1-chloro-3-hydroxypropane is high yielding (95%-100%) and the use of acrylonitrile, with its attendant disadvantages, is thereby avoided. The 3-(4-fluorophenoxy)-1-propanol so produced is thereafter easily converted to 3-(4-fluorophenoxy)-propionic acid by means of oxidation with potassium permanganate in a new step (2), and the latter intermediate is then condensed with concentrated sulfuric acid to yield the desired 6-fluoro-4-chromanone.

In accordance with the process employed for preparing the novel compound of this invention, 4-fluorophenol is contacted in a polar protic solvent with a 1-halo-3-hydroxypropane compound of the formula $XCH_2CH_2CH_2OH$, wherein X is either chlorine or bromine, in the presence of a base that serves as a hydrohalide acceptor. Preferred solvents for use in this connection include water or a lower alkanol such as methanol, ethanol or isopropanol, while preferred bases include alkali metal hydroxides such as sodium hydroxide, potassium hydroxide and the like, as well as organic amines such as tertiary amines like triethylamine and pyridine, etc. In general, substantially equimolar amounts of reactant and reagent are employed (i.e., from about 0.80 to about 1.25 mole of halide reagent with respect to 4-fluorophenol starting material) and the reaction is normally effected at a temperature that is in the range of from about 40°C up to about 100°C for a period of about 15 minutes to about eight hours. Preferably, the reaction is carried out using a slight excess of alkylating agent in an aqueous medium at a temperature near or just below the reflux temperature of the reaction mixture for a period of time that is ordinarily less than about four hours. Upon completion of the reaction, the desired product is readily isolated from the reaction mixture in a conventional manner, e.g., by extraction with a suitable organic solvent such as a water-immiscible halogenated hydrocarbon like methylene chloride, chloroform, ethylene dichloride and S-tetrachlorethane, etc.

Conversion of the desired 3-(4-fluorophenoxy)-1-propanol compound, prepared as described above, to 3-(4-fluorophenoxy)propionic acid is then effected in a most facile manner, viz., by oxidizing the aforesaid alcohol with potassium permanganate in aqueous acetic acid at room temperature. This method represents a modification of the synthesis of 3-phenoxypropionic acid described by S.G. Powell in the Journal of the American Chemical Society, Vol. 45, p. 2708 (1923). However, the latter publication reported that the oxidation reaction was limited in scale to less than 10 g and the yield was below 45%, whereas the present study has found that by controlling the pH of the reaction at ca. pH 5, preferably by the addition of glacial acetic acid, an improvement in yields is readily obtained.

As previously indicated, 3-(4-fluorophenoxy)-propionic acid is then easily converted to 6-fluoro-4-chromanone by condensing the organic acid in the presence of concentrated sulfuric acid according to step (3) of the prior art.

The starting materials required for preparing the novel product of this invention are all known compounds.

*Example 1*

To a solution consisting of 4-fluorophenol (33.6 g, 0.3 mole) and sodium hydroxide (13 g, 0.325 mole) dissolved in water (100 ml) at 40-50°C, there was added 1-chloro-3-hydroxypropane (32.7 g, 0.345 mole). The resulting reaction solution was heated to 93°C on a steam bath for a period of three hours, during which time some of the product separated as an oil. At the end of this time, the resulting reaction mixture was allowed to cool to room temperature (~20°C). The oil was then separated from the aqueous layer, and the latter was extracted with methylene chloride (2 × 100 ml). The organic layers were combined and subsequently washed with water (2 × 70 ml) and thereafter dried over anhydrous sodium sulfate. After removal of the drying agent by means of filtration and the solvent by means of evaporation under reduced pressure, there was finally obtained the desired product, viz., 3-(4-fluorophenoxy)-1-propanol, as an oil concentrate. The latter product was used as such in the next reaction step without any further purification being necessary. The yield of product was 98% of the theoretical value. The product was characterized by the following nuclear magnetic resonance data: NMR /CDCl$_3$) Δ 7.1 (m, 4), 4.1 (t, 2), 3.9 (t, 2), 3.0 (broad s, 1), 2.0 (m, 2).

*Example 2*

To a stirred mixture consisting of 3-(4-fluorophenoxy)-1-propanol (8.5 g, 0.05 mole) and water (100 ml) at 20°C, there was added in a dropwise manner over a period of one-hour a solution consisting of potassium permanganate (17.3 g, 0.11 mole) dissolved in water (262 ml) containing acetic acid (7.2 g, 0.12 mole). After stirring the resulting reaction mixture for a period of one hour, the pH was lowered from 4.8 to 1.5 by the addition of concentrated hydrochloric acid (11 ml). Sodium bisulfite (14 g, 0.35 mole) was then added to the mixture in portions over a 15-minute period, while the pH was maintained at 1.5 by the addition of concentrated hydrochloric acid (24 ml). The resulting white slurry of product was then collected by means of suction filtration and the desired material subsequently washed with water and air-dried. In this way, there were ultimately obtained 5.3 g (58%) of pure 3-(4-fluorophenoxy)propionic acid. This material was identical to the product obtained by the hydrolysis of 3-(4-fluorophenoxy)propionitrile. It was also identical with the product first reported by G. C. Finger et al. in the Journal of the American Chemical Society, Vol. 81, p. 94 (1959).

*Example 3*

3-(4-Fluorophenoxy)propionic acid (30 g, 0.163 mole) was added in portions to concentrated sulfuric acid (90 ml) held at 50°C with stirring. The resulting reaction mixture was then stirred at this same temperature for a period of 15 minutes after the addition was complete prior to being slowly poured into a stirred ice-water slurry (400 ml). The desired product then precipitated as a white solid, was granulated for two hours and finally collected by filtration and dried in vacuo to ultimately yield 22.1 g, (82%) of pure 6-fluoro-4-chromanone, m.p. 111-113°C. This material was identical to the product first reported by R. Sarges in U.S. Patent No. 4 117 230.

*Example 4*

The procedure described in Example 1 is repeated except that 1-bromo-3-hydroxypropane is the reagent employed instead of 1-chloro-3-hydroxypropane, using the same molar proportions as before. In this particular case, the corresponding final product obtained is also 3-(4-fluorophenoxy)-1-propanol, identical to the product of Example 1.

**Claim**

1. 3-(4-Fluorophenoxy)-1-propanol.

**Patentanspruch**

1. 3-(4-Fluorophenoxy)-1-propanol.

**Revendication**

1. 3-(4-fluorophénoxy)-1-propanol.